# EUROPEAN PATENT APPLICATION

(11) **EP 4 015 500 A1**
(43) Date of publication of application: **22.06.2022**
(21) Application number: 20215315.1
(22) Date of filing: 18.12.2020
(51) Int. Cl.: C07C 227/18, C07C 227/10, C07C 227/40

(54) **PROCESS OF MANUFACTURING 2-(4 -DIETHYLAMINO-2 -HYDROXYBENZOYL)BENZOIC ACID HEXYL ESTER**

(71) Applicant: BASF SE, 67056 Ludwigshafen am Rhein (DE)
(72) Inventor: The designation of the inventor has not yet been filed
(74) Representative: BASF IP Association

(57) **Abstract**

The present invention relates to a process of manufacturing 2-(4'-diethylamino-2'-hydroxybenzoyl)benzoic acid hexyl ester having a Gardner value of 8.2 or less comprising adjusting the reaction mixture obtained by reacting 2-(4'-diethylamino-2'-hydroxybenzoyl)benzoic acid with n-hexanol to a pH of more than 7 followed by extracting at least once with an aqueous phase and adsorbing the dissolved 2-(4'-diethylamino-2'-hydroxybenzoyl)benzoic acid hexyl ester over at least two different adsorbents.

## Description

The present invention relates to a process for manufacturing 2-(4'-diethylamino-2'-hydroxybenzoyl)benzoic acid hexyl ester having a low Gardner value.

UV radiation causes harmful effects on the human skin. Beside the acute effect of sunburn of the skin, UV radiation is also known to increase the risk of skin cancer. Furthermore, long time exposure to UV-A and UV-B light can cause phototoxic and photo allergenic reactions on the skin and can accelerate skin aging.

To protect the human skin from UV radiation, various sun protecting UV filters (also referred to as UV absorbers) exist including UV-A filter, UV-B filter, and broadband filters. These filters are added to sunscreen or cosmetic compositions. The UV filters are either organic or inorganic, particulate or non-particulate compounds, of which all have a high absorption efficacy in the UV-light range. In general, UV light can be divided into UV-A radiation (320 - 400 nm) and UV-B radiation (280 - 320 nm). Depending on the position of the absorption maxima, UV filters are divided into UV-A and UV-B filters. In case an UV filter absorbs both, UV-A and UV-B light, it is referred to as a broadband absorber.

Since 2006, the EU commission has recommended that all sunscreen or cosmetic compositions should have an UV-A protection factor, which is at least one third of the labelled sun protection factor (SPF), wherein the sun protection factor refers mainly to the UV-B protection.

2-(4'-Diethylamino-2'-hydroxybenzoyl)benzoic acid hexyl ester is an effective UV-A filter and is used to protect skin from UV radiation (e.g. in sunscreen). 2-(4'-Diethylamino-2'-hydroxybenzoyl)benzoic acid hexyl ester is depicted below, and will in the following also be referred to as DHHB or compound of formula (I).

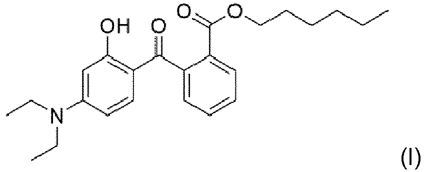

Processes of manufacturing 2-(4'-diethylamino-2'-hydroxybenzoyl)benzoic acid hexyl ester are known from e.g. DE 10011317, EP2155660, and WO 2003097578. In this connection, several challenges have been observed.

During the synthesis of 2-(4'-diethylamino-2'-hydroxybenzoyl)benzoic acid hexyl ester process Rhodamine B type dyes (e.g. [9-(2-carboxyphenyl)-6-diethylamino-3-xanthenylidene]-diethylammonium salts) and corresponding esters are formed as impurities, which lead to an undesired discoloration of the final product. In order to purify the final product (less than 5 ppm Rhodamine B type dyes) an additional purification step is required. So far, the purification step comprises dissolution of the contaminated 2-(4'-diethylamino-2'-hydroxybenzoyl)benzoic acid hexyl ester in a nonpolar solvent, adsorption over an adsorbent and evaporation of the solvent after passage through the bed.

After the subsequent isolation of the 2-(4'-diethylamino-2'-hydroxybenzoyl)benzoic acid hexyl ester by separating off solvent by distillation the clean end product is bottled as melt and optionally further solidified according to EP2155660.

Hence, there is an ongoing need for a fast and efficient process of manufacturing of 2-(4'-diethylamino-2'-hydroxybenzoyl)benzoic acid hexyl ester having particularly reduced color impurities. It has in particularly been an object to reduce color impurities, which results in undesired reddish and brownish discoloration of 2-(4'-diethylamino-2'-hydroxybenzoyl)benzoic acid hexyl ester. Further, it has been an object of the present invention to provide an efficient, economically improved, process for isolating crystalline 2-(4'-diethylamino-2'-hydroxybenzoyl)benzoic acid hexyl ester.

It has surprisingly been found that at least one of the above objects can be achieved by the process according to the present invention. The inventive process provides 2-(4'-diethylamino-2'-hydroxybenzoyl)benzoic acid hexyl ester with low rhodamine content (less than 5 ppm and in particular less than 1 ppm) and low content of potential further non-reddish colored impurities.

In particular, the inventors of the present invention have found a process of effectively manufacturing the compound of formula (I).

The present invention therefore relates in a first aspect to a process of manufacturing 2-(4'-diethylamino-2'-hydroxybenzoyl)benzoic acid hexyl ester comprising the steps of
a) reacting phthalic acid anhydride with 3-N,N-diethylaminophenol to obtain 2-(4'-diethylamino-2'-hydroxybenzoyl)benzoic acid;
b) reacting the 2-(4'-diethylamino-2'-hydroxybenzoyl)benzoic acid obtained in step a) with n-hexanol to obtain 2-(4'-diethylamino-2'-hydroxybenzoyl)benzoic acid hexyl ester;
c) adjusting the reaction mixture obtained in step b) to a pH of more than 7 followed by extracting at least once with an aqueous phase; and
d) adsorbing the dissolved 2-(4'-diethylamino-2'-hydroxybenzoyl)benzoic acid hexyl ester over at least two different adsorbents.

In the following, preferred embodiments of the process of the first aspect are described in further detail. It is to be understood that each preferred embodiment is relevant on its own as well as in combination with other preferred embodiments.

In a preferred embodiment A1 of the first aspect, the 3-N,N-diethylaminophenol applied in step a) is distilled prior reacting with the phthalic acid anhydride and/or has a Gardner value of 20 or lower, a Yellowness Index of 80 or lower, and/or a Hess-Ives of 350 or lower.

In a preferred embodiment A2 of the first aspect, the pH of the reaction mixture in the extraction step c) is adjusted with an aqueous alkaline solution.

In a preferred embodiment A3 of the first aspect, the pH of the reaction mixture in extraction step c) is from 8 to 14, preferably from 9 to 13, and in particular from 10.5 to 12.5.

In a preferred embodiment A4 of the first aspect, in step c) the reaction mixture obtained in step b) is adjusted to a pH or more than 7 by the addition of a base to the reaction mixture obtained in step b).

In a preferred embodiment A5 of the first aspect, the process further comprises the step cll) crystallizing the 2-(4'-diethylamino-2'-hydroxybenzoyl)benzoic acid hexyl ester from the organic phase obtained in the extraction step c).

In a preferred embodiment A6 of the first aspect, in the crystallization step cll), the crystallized 2-(4'-diethylamino-2'-hydroxybenzoyl)benzoic acid hexyl ester is dissolved in an organic solvent prior to the adsorption step d), preferably wherein the crystallized 2-(4'-diethylamino-2'-hydroxybenzoyl)benzoic acid hexyl ester is dissolved in a non-polar solvent, more preferably in toluene.

In a preferred embodiment A7 of the first aspect, the at least two different adsorbents are selected from the group consisting of activated carbons, aluminum oxides, zeolites, and silica gels, preferably activated carbons and silica gels.

In a preferred embodiment A8 of the first aspect, in the adsorption step d) the dissolved 2-(4'-diethylamino-2'-hydroxybenzoyl)benzoic acid hexyl ester is first adsorbed over silica gel and then adsorbed over activated carbon.

In a preferred embodiment A9 of the first aspect, the solution in adsorption step d) comprises 2-(4'-diethylamino-2'-hydroxybenzoyl)benzoic acid hexyl ester from 10 to 80 wt.-%, preferably from 20 to 60 wt.-%, and in particular from 25 to 40 wt.-%, based on the total weight of the solution in adsorption step d).

In a preferred embodiment A10 of the first aspect, the esterification step b) is carried out in the presence of an acidic catalyst, preferably wherein the acidic catalyst is sulfuric acid.

In a preferred embodiment A11 of the first aspect, the process further comprises the step of
e) removing the solvent, preferably wherein the solvent is removed by distillation.

In a preferred embodiment A12 of the first aspect, the manufactured 2-(4'-diethylamino-2'-hydroxybenzoyl)benzoic acid hexyl ester has a Gardner value of 8.2 or less, a Yellowness Index of less than 89, a Hess-Ives of less than 19, and/or comprises less than 5 ppm, preferably less than 2 ppm, and in particular less than 1 ppm, of rhodamine and its esters.

In a second aspect, the invention relates to 2-(4'-diethylamino-2'-hydroxybenzoyl)benzoic acid hexyl ester having a Gardner value of 8.2 or less, preferably less than 7.9, and in particular less than 7.5, and comprising less than 5 ppm, preferably less than 2 ppm, and in particular less than 1 ppm, of rhodamine and its esters, preferably wherein the 2-(4'-diethylamino-2'-hydroxybenzoyl)benzoic acid hexyl ester is obtained by the process according to first aspect.

In a preferred embodiment B1 of the second aspect, the 2-(4'-diethylamino-2'-hydroxybenzoyl)benzoic acid hexyl ester further has a Yellowness Index of less than 89, preferably less than 86 and/or a Hess-Ives of less than 19, preferably less than 17.

### Detailed Description

Before describing in detail exemplary embodiments of the present invention, definitions important for understanding the present invention are given.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below. It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

The term "compound according to the invention" or "compound of formula (I)" refers to 2-(4'-diethylamino-2'-hydroxybenzoyl)benzoic acid hexyl ester.

The term "halogen" denotes in each case fluorine, bromine, chlorine, or iodine, in particular fluorine, chlorine, or bromine.

The term "body-care product" refers to any product suitable to apply to the human body, e.g. sunscreen compositions, bath and shower products, preparations containing fragrances and odoriferous substances, hair-care products, dentifrices, decorative preparations, and cosmetic formulations containing active ingredients. Preferred are sunscreen compositions.

The term "sunscreen composition" or "sunscreen" or "skin-care product" refers to any topical product, which reflects and/or absorbs certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, foams, powders, or gels. According to the present invention the sunscreen composition may comprise one or more active agents, e.g., organic UV filters, as well as other ingredients or additives, e.g., emulsifiers, emollients, viscosity regulators, stabilizers, preservatives, or fragrances.

The term "daily care composition" refers to any topical product, which reflects or absorbs certain parts of UV radiation and is used as an everyday care product for the human body, e.g., for face, body or hair. The daily care composition may comprise one or more active agents, e.g., organic or inorganic UV filters, as well as other ingredients or additives, e.g., emulsifiers, emollients, viscosity regulators, stabilizers, preservatives, or fragrances.

Suitable bath and shower additives are, e.g., shower gels, bath-salts, bubble baths and soaps.

Suitable preparations containing fragrances and odoriferous substances are in particular scents, perfumes, toilet waters and shaving lotions (aftershave preparations).

Suitable hair-care products are, e.g., shampoos for humans and animals, in particular dogs, hair conditioners, products for styling and treating hair, perming agents, hair sprays and lacquers, hair gels, hair fixatives and hair dyeing or bleaching agents.

Suitable dentifrices are, e.g., tooth creams, toothpastes, mouth-washes, mouth rinses, antiplaque preparations and cleaning agents for dentures.

Suitable decorative preparations are, e.g., lipsticks, nail varnishes, eye shadows, mascaras, dry and moist make-up, rouge, powders, depilatory agents and suntan lotions.

Suitable cosmetic formulations containing active ingredients are, e.g., hormone preparations, vitamin preparations, vegetable extract preparations and antibacterial preparations.

The cited body-care products can be in the form of creams, ointments, pastes, foams, gels, lotions, powders, make-ups, sprays, sticks or aerosols. Thereby, the compound of formula (I) may acts as a light stabilizer.

The term "critical wavelength" is defined as the wavelength at which the area under the UV protection curve (% protection versus wavelength) represents 90 % of the total area under the curve in the UV region (280-400 nm). For example, a critical wavelength of 370 nm indicates that the protection of the sunscreen composition is not limited to the wavelengths of UV-B, i.e. wavelengths from 280-320 nm, but extends to 370 nm in such a way that 90 % of the total area under the protective curve in the UV region are reached at 370 nm.

The term "ultraviolet filter" or "UV filter" as used herein refers to organic or inorganic compounds, which can absorb and/or reflect UV radiation caused by sunlight. UV filter can be classified based on their UV protection curve as UV-A, UV-B or broadband filters. In the context of the present application, broadband filters may be listed as UV-A filters, as they also provide UV-A protection. In other words, preferred UV-A filters also include broadband filters.

The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. Furthermore, it is recommended by the European Commission that all sunscreen or cosmetic compositions should have an UVA protection factor, which is at least one third of the labelled sun protection factor (SPF), e.g. if the sunscreen composition has an SPF of 30 the UVA protection factor has to be at least 10.

Preferred embodiments regarding the process according to the present invention are described hereinafter. It is to be understood that the preferred embodiments of the invention are preferred alone or in combination with each other.

As indicated above, the present invention relates in one embodiment to a process of manufacturing 2-(4'-diethylamino-2'-hydroxybenzoyl)benzoic acid hexyl ester comprising the steps of
a) reacting phthalic acid anhydride with 3-N,N-diethylaminophenol to obtain 2-(4'-diethylamino-2'-hydroxybenzoyl)benzoic acid;
b) reacting the 2-(4'-diethylamino-2'-hydroxybenzoyl)benzoic acid obtained in step a) with n-hexanol to obtain 2-(4'-diethylamino-2'-hydroxybenzoyl)benzoic acid hexyl ester;
c) adjusting the reaction mixture obtained in step b) to a pH of more than 7 followed by extracting at least once with an aqueous phase; and
d) adsorbing the dissolved 2-(4'-diethylamino-2'-hydroxybenzoyl)benzoic acid hexyl ester over at least two different adsorbents.

Preferred embodiments regarding the process are defined hereinafter.

In a preferred embodiment of the present invention, the 3-N,N-diethylaminophenol applied in step a) is distilled prior reacting with the phthalic acid anhydride. Distillation may be achieved by any known in the art method.

Preferably, distillation of the 3-N,N-diethylaminophenol applied in step a) is done within 72 hours, more preferably within 48 hours, even more preferred within 24 hours, and in particular within 12 hours prior the reaction according to step a). In a particular preferred embodiment, the distillation of the 3-N,N-diethylaminophenol applied in step a) is done within six hours, more preferably within 3 hours, even more preferably within 1 hour, still more preferably within 30 minutes, and in particular within 5 minutes, prior the reaction according to step a).

In a preferred embodiment of the present invention, the applied 3-N,N-diethylaminophenol applied in step a) has a Gardner value (determined according to ASTM D1544-04) of 20 or lower, more preferably 18 or lower, even more preferably 15 or lower, still more preferably 10 or lower, and in particular 7 or lower.

In another preferred embodiment of the present invention, the applied 3-N,N-diethylaminophenol applied in step a) has a Yellowness Index (determined according to ASTM D 5386-93b based on CIE XYZ-tristimulus values, standard illuminant C and a 2° standard observer) of 80 or lower, more preferably of 70 or lower, even more preferably of 60 or lower, still more preferably of 50 or lower, and in particular of 40 or lower.

In another preferred embodiment of the present invention, the applied 3-N,N-diethylaminophenol applied in step a) has a Hess-Ives-value (determined according to DGK method no. F 050.2) of 350 or lower, more preferably of 280 or lower, even more preferably of 150 or lower, still more preferably of 50 or lower, and in particular or 20 or lower.

Preferably, the applied 3-N,N-diethylaminophenol applied in step a) has a Gardner value (determined according to ASTM D1544-04) of 20 or lower, a Yellowness Index (determined according to ASTM D 5386-93b based on CIE XYZ-tristimulus values, standard illuminant C and a 2° standard observer) of 80 or lower, and a Hess-Ives-value (determined according to DGK method no. F 050.2) of 350 or lower.

In a preferred embodiment of the present invention, the esterification step b) is carried out in the presence of an acidic catalyst. Suitable acid catalysts are, for example, hydrogen chloride, sulfuric acid, nitric acid, phosphoric acid, sulfonic acids, such as benzenesulfonic acid, p-toluenesulfonic acid, methanesulfonic acid or mixtures of these acids, but also sulfonic acid group-containing ion exchangers, such as, for example, Lewatits S100 (Bayer). Preferred acidic catalysts are hydrogen chloride, sulfuric acid, methanesulfonic acid, and p-toluenesulfonic acid, in particular sulfuric acid.

Preferably, the reactions according to steps a) and b) are performed in a solvent. The solvent may be selected from the group consisting of aromatic hydrocarbons such as benzene, toluene, and xylene(s); aliphatic C5-C12-hydrocarbons such as octane, petroleum ether, isooctane, and decane; ethers such as diethyl ether, dibutyl ether, and tetrahydrofuran; ketones such as acetone and acetophenone; esters such as ethyl acetate and isopropyl acetate; alcohols such as methanol, ethanol, isopropanol, and hexanol; chlorinated hydrocarbons such as perchloroethylene and chlorobenzene; cyclohexane; and mixtures thereof. Particularly preferred solvents are for step a) toluene and xylene(s) and, for step b), hexanol.

In step c), the pH may be adjusted with any suitable base known is the art. Preferably, the pH is adjusted with an alkaline solution derived from alkali metal and alkaline earth metal hydroxides, alkali metal and alkaline earth metal oxides, alkali metal and alkaline earth metal carbonates, alkali metal and alkaline earth metal hydrogen carbonates, alkali metal and alkaline earth metal alcoholates, and nitrogen containing bases including tertiary amines, pyridines, and bicyclic amines. In particular, the pH is adjusted with an aqueous alkaline solution.

A suitable aqueous alkaline solution may be an aqueous alkaline solution which comprises at least one hydroxide of at least one metal selected from the group consisting of alkali metals and alkaline earth metals. Preferred are aqueous sodium hydroxide and aqueous potassium hydroxide solutions. Preferably, the aqueous alkaline solution is a 0.5 to 5%, more preferably 1 to 4%, and in particular 2 to 3%, aqueous sodium hydroxide solution.

In a preferred embodiment of the present invention, the pH of the reaction mixture in extraction step c) is from 8 to 14, more preferably from 9 to 13, and in particular from 10.5 to 12.5. Alternatively, water is added to the reaction mixture obtained in step b) and the pH is adjusted by addition of a higher concentrated base, e.g. 25% aqueous sodium hydroxide or 25% aqueous potassium hydroxide solutions. In this connection, the addition of the higher concentrated base is stopped when a pH of more than 7, preferably from 8 to 14, more preferably from 9 to 13, and in particular from 10.5 to 12.5, is reached.

In a preferred embodiment of the present invention, in step c) the reaction mixture obtained in step b) is adjusted to a pH of more than 7 by the addition of a base to the reaction mixture obtained in step b). In this connection it is to be understood that the reaction mixture obtained in step b) is not further diluted with an organic solvent before adjusting the pH to a pH of more than 7.

In a preferred embodiment of the present invention, the process further comprises the step
cll) crystallizing the 2-(4'-diethylamino-2'-hydroxybenzoyl)benzoic acid hexyl ester from the organic phase obtained in the extraction step c).

Preferably, in the crystallization step cll) the crystallized 2-(4'-diethylamino-2'-hydroxybenzoyl)benzoic acid hexyl ester is dissolved in an organic solvent prior to the adsorption step d).

Suitable organic solvents applied in step cll) to dissolve the crystallized 2-(4'-diethylamino-2'-hydroxybenzoyl)benzoic acid hexyl ester are ketones; ethers; esters such as ethyl acetate and isopropyl acetate; alcohols such as methanol, ethanol, isopropanol, and n-hexanol; non-polar aliphatic and aromatic hydrocarbons such as toluene, xylene(s), cyclohexane, and petroleum ether; and mixtures thereof.

Preferably, the crystallized 2-(4'-diethylamino-2'-hydroxybenzoyl)benzoic acid hexyl ester is dissolved in a non-polar solvent.

It is to be understood that a non-polar solvent has a dielectric constant (ε) of less than 15. The non-polarity may alternatively or additionally be expressed by a dipole moment (µ) of from 0 to 2. In this connection it is particularly preferred when the non-polar solvent is selected from the group consisting of petroleum ether, ligroin, n-hexane, cyclohexane, heptane, di-n-butyl ether, xylene, toluene, benzene, and mixtures thereof.

According to a particular preferred embodiment of the present invention, the crystallized 2-(4'-diethylamino-2'-hydroxybenzoyl)benzoic acid hexyl ester is dissolved in toluene.

In adsorption step d), the dissolved 2-(4'-diethylamino-2'-hydroxybenzoyl)benzoic acid hexyl ester is preferably dissolved in an organic solvent. The organic solvent is preferably selected form the group consisting of ketones; ethers; esters such as ethyl acetate and isopropyl acetate; alcohols such as methanol, ethanol, isopropanol, and n-hexanol; non-polar aliphatic and aromatic hydrocarbons such as toluene, xylene(s), cyclohexane, and petroleum ether; and mixtures thereof. More preferably, the dissolved 2-(4'-diethylamino-2'-hydroxybenzoyl)benzoic acid hexyl ester is dissolved in a non-polar solvent. In particular, the solvent in the adsorption step d) is selected from the group consisting of toluene, xylene(s), and mixtures thereof.

In a preferred embodiment of the present invention, the at least two different adsorbents are selected from the group consisting of activated carbons, aluminum oxides, zeolites, and silica gels, preferably activated carbons and silica gels. In a more preferred embodiment of the present invention, the at least two different adsorbents differ in the chemical composition, i.e. that if the first adsorbent is an activated carbon, the second adsorbent is selected from the group consisting of aluminum oxides, zeolites, and silica gels, preferably activated carbons and silica gels.

In the event that activated carbon and silica gel are used as the at least two adsorbents, they can be applied in any ratio. Preferably they are applied in a weight ratio activated carbon to silica gel of 0.001 to 2, more preferably of 0.005 to 1.5, even more preferably of 0.01 to 1.0, and in particular of 0.02 to 0.5.

The silica gels suitable as adsorbents are described, inter alia, in the CD Rompp Chemie Lexikon-Version 1.0, keyword: silica gels, Stuttgart/New York: Georg Thieme Verlag 1995 and the literature cited therein. Preferred silica gels are silica gel 60 from Merck, Darmstadt and silica gel 123 from Grace.

Activated carbon may be used in powder form, granule form or as cylindrically formed particles. In this connection, the activated carbon is advantageously used in granule form (granular activated carbon) in fixed- or fluidized-bed filters. Examples of preferred carbons are the activated carbons CPG(R) LF, CAL(R) and APC(R) from Chemviron Carbon or Norit C Gran and Norit GAC 1240 Plus from Cabot or Acticarbone BGE from Ceca.

Further details on properties and grades of the activated carbons used are given in Ullmann's Encyclopedia, Sixth Edition, 2000 Electronic Release, Chapter 5.

In a preferred embodiment of the present invention, in the adsorption step d) the dissolved 2-(4'-diethylamino-2'-hydroxybenzoyl)benzoic acid hexyl ester is first adsorbed over silica gel and then adsorbed over activated carbon.

In a still preferred embodiment of the present invention, in the adsorption step d) the dissolved 2-(4'-diethylamino-2'-hydroxybenzoyl)benzoic acid hexyl ester is first adsorbed over silica gel, then adsorbed over activated carbon and then adsorbed over silica gel again.

In another preferred embodiment of the present invention, in the adsorption step d) the dissolved 2-(4'-diethylamino-2'-hydroxybenzoyl)benzoic acid hexyl ester is first adsorbed over activated carbon and then adsorbed over silica gel, preferably followed by a further adsorption over activated carbon.

Preferably, the adsorption step d) is performed at a temperature in the range of from 15 to 80 °C, more preferably from 18 to 70 °C, even more preferably from 19 to 60 °C, still more preferably from 20 to 50 °C, and in particular from 20 to 25°C.

Preferably, the solution in adsorption step d) comprises 2-(4'-diethylamino-2'-hydroxybenzoyl)benzoic acid hexyl ester from 10 to 80 wt.-%, more preferably from 20 to 60 wt.-%, even more preferably from 22 to 50 wt.-%, and in particular from 25 to 40 wt.-%, based on the total weight of the solution in adsorption step d).

Preferably, the adsorption is conducted continuously in a fixed bed (column). However, it can also be conducted discontinuous by addition of the adsorbents to a solution of 2-(4'-diethylamino-2'-hydroxybenzoyl)benzoic acid hexyl ester in a solvent, preferably in an organic solvent as described above, followed by stirring and filtration of the adsorbents.

In a preferred embodiment of the present invention, the process further comprises the step of
e) removing the solvent, preferably wherein the solvent is removed by distillation.

It is to be understood that step e) is conducted after the adsorption step d). In order to remove potentially remaining solvent after step e), it is preferred to conduct an additional stripping step.

In a preferred embodiment of the present invention, the manufactured 2-(4'-diethylamino-2'-hydroxybenzoyl)benzoic acid hexyl ester has a Gardner value of 8.2 or less, preferably less than 7.9, more preferably less than 7.6, and in particular less than 7.5, a Yellowness Index of less than 89, preferably less than 86, more preferably less than 84, and in particular less than 82, a Hess-Ives of less than 19, preferably less than 17, more preferably less than 16, and in particular less than 15, and/or comprises less than 5 ppm, preferably less than 2 ppm, and in particular less than 1 ppm, of rhodamine and its esters.

In general, it is assumed that the Gardner value is particularly suitably to express brownish color impurities so that a low Gardner value indicates a low brownish discoloration. Undesired reddish color impurities of the final product 2-(4'-diethylamino-2'-hydroxybenzoyl)benzoic acid hexyl ester are preferably expressed using Hess-Ives so that a low Hess-Ives indicates a low reddish discoloration.

In a second aspect, the invention relates to 2-(4'-diethylamino-2'-hydroxybenzoyl)benzoic acid hexyl ester having a Gardner value of 8.2 or less, preferably less than 7.9, more preferably less than 7.6, and in particular less than 7.5 and comprising less than 5 ppm, preferably less than 2 ppm, and in particular less than 1 ppm, of rhodamine and its esters.

Preferably, the 2-(4'-diethylamino-2'-hydroxybenzoyl)benzoic acid hexyl ester further has a Yellowness Index of less than 89, preferably less than 86, more preferably less than 84, and in particular less than 82 and/or a Hess-Ives of less than 19, preferably less than 17, more preferably less than 16, and in particular less than 15.

Preferably, the invention relates to 2-(4'-diethylamino-2'-hydroxybenzoyl)benzoic acid hexyl ester having a Gardner value of 8.2 or less, preferably less than 7.9, more preferably less than 7.6, and in particular less than 7.5, a Yellowness Index of less than 89, preferably less than 86, more preferably less than 84, and in particular less than 82, a Hess-Ives of less than 19, preferably less than 17, more preferably less than 16, and in particular less than 15, and comprising less than 5 ppm, preferably less than 2 ppm, and in particular less than 1 ppm, of rhodamine and its esters.

In a preferred embodiment, the 2-(4'-diethylamino-2'-hydroxybenzoyl)benzoic acid hexyl ester having a Gardner value of 8.2 or less, preferably less than 7.9, more preferably less than 7.6, and in particular less than 7.5 and comprising less than 5 ppm, preferably less than 2 ppm, and in particular less than 1 ppm, of rhodamine and its esters is obtained by the process according to first aspect. Preferably, the 2-(4'-diethylamino-2'-hydroxybenzoyl)benzoic acid hexyl ester obtained by the process according to first aspect further has a Yellowness Index of less than 89, preferably less than 86, more preferably less than 84, and in particular less than 82 and/or a Hess-Ives of less than 19, preferably less than 17, more preferably less than 16, and in particular less than 15.

In a particular preferred embodiment, the 2-(4'-diethylamino-2'-hydroxybenzoyl)benzoic acid hexyl ester having a Gardner value of 8.2 or less, preferably less than 7.9, more preferably less than 7.6, and in particular less than 7.5, a Yellowness Index of less than 89, preferably less than 86, more preferably less than 84, and in particular less than 82, a Hess-Ives of less than 19, preferably less than 17, more preferably less than 16, and in particular less than 15, and comprising less than 5 ppm, preferably less than 2 ppm, and in particular less than 1 ppm, of rhodamine and its esters is obtained by the process according to first aspect.

The 2-(4'-diethylamino-2'-hydroxybenzoyl)benzoic acid hexyl ester obtained according to the claimed process may be comprised in a body-care product or a daily care composition. In particular, the 2-(4'-diethylamino-2'-hydroxybenzoyl)benzoic acid hexyl ester obtained according to the claimed process may be comprised in sunscreen compositions.

The 2-(4'-diethylamino-2'-hydroxybenzoyl)benzoic acid hexyl ester obtained according to the claimed process may be used as UV filter in a body-care product or a daily care composition, in particular in a sunscreen composition.

The present invention is further illustrated by the following examples.

### Experimental part

### Methods

HPLC method for determination of 2-(4'-diethylamino-2'-hydroxybenzoyl)benzoic acid hexyl ester and rhodamine type impurities

HPLC Chromatograph with DAD/UV-detector (Agilent 1100 or similar). HPLC-chromatographic column: Symmetry C18 5 µm 250 x 4.6 mm Waters

Solvent A: Water + 0,1% v/v o-phosphoric acid 85%, Solvent B: Acetonitrile. Flow rate 1.2 ml/min. Injection volume 5 µL, T=20°C.

Gradient: t=0 min 80% A, 20% B /t=20 min, 0% A, 100% B /t=30 min, 0% A, 100% B /t=32 min, 80% A, 20% B.

Rhodamine B: RT = 8.8 min (558 nm), rhodamine-hexylester: RT = 11.9 min (558 nm), 2-(4'-diethylamino-2'-hydroxybenzoyl)benzoic acid hexyl ester: RT = 21.7 min (360 nm).

External standards: Rhodamine B, rhodamine-hexylester, 2-(4'-diethylamino-2'-hydroxybenzoyl)benzoic acid hexyl ester.

### Color assessment

Color values were determined by a Lico 690 colorimeter (Hach-Lange) using 11 mm round cuvettes. Samples were prepared by heating 2-(4'-diethylamino-2'-hydroxybenzoyl)benzoic acid hexyl ester to 65°C, homogenization and transfer to the cuvette. The color value was measured after 5-7 min of cooling.

The Gardner value has been determined according to ASTM D1544-04.

The Yellowness Index has been determined according to ASTM D 5386-93b based on CIE XYZ-tristimulus values, standard illuminant C and a 2° standard observer.

The Hess-Ives-value has been determined according to DGK method no. F 050.2.

Analytical standards for calibration have been performed by standard state of the art methods.

Silica 123 (Grace) was used as silica adsorbent.

Norit C GRAN (Cabot Norit) was used as activated carbon adsorbent.

### Examples

### General procedure

2-(4'-Diethylamino-2'-hydroxybenzoyl)benzoic acid hexyl ester (DHHB) was synthesized based on the process described in WO 03097578 (examples 1 and 2). Some parameters (Table 2) were varied; i.e. i) 3-N,N-Diethylaminophenol (DEMAP) was distilled/not distilled before use and/or ii) the pH during extraction was set to pH of 7 or to pH of approximately 11.5. The process comprises the following general steps:
General step 1: Synthesis of 2-(4'-diethylamino-2'-hydroxybenzoyl)benzoic acid 3-N,N-Diethylaminophenol (DEMAP) was reacted with phthalic acid anhydride in toluene at reflux followed by isolation of 2-(4'-Diethylamino-2'-hydroxybenzoyl)benzoic acid by filtration and washing of the filter cake with hexanol. The wet filter cake was used for the second step.
General step 2: Synthesis of 2-(4'-diethylamino-2'-hydroxybenzoyl)benzoic acid hexyl ester Esterification of the acid was performed in hexanol at reflux with azeotropic removal of water in the presence an acidic catalyst, preferably sulfuric acid, yielding 2-(4'-Diethylamino-2'-hydroxybenzoyl)-benzoic acid hexyl ester. After reaction, the pH of the reaction mixture was adjusted to the pH given in the examples underneath using 2.3% aqueous sodium hydroxide. The organic phase was washed once more with water and the ester was crystallized from the organic phase after extraction. The filter cake was washed with hexanol and the wet filter cake used for adsorption.

### DEMAP Distillation

The distillation was done at low pressure using common distillation equipment, e.g. a distillation bridge or a short path distillation (thin film evaporator). A distillation column is suited as well.

### Distillation (bridge):

450 g DEMAP (dark brown, 97.1 wt.-% by HPLC) were distilled over a Claisen bridge at 160-170 °C (heating jacket) / 0.7 mbar. Four fractions were taken: 1) 92.1 g; 2) 18.4 g; 3) 7.7 g and 4) 330.6 g. Fraction 1-3 were light yellow, fraction 4 was yellow. The fourth fraction with a DEMAP content of 99.1 wt.-% (HPLC) was used for the synthesis of DHHB.

### Example for thin film evaporation:

353.5 g DEMAP (94.2 wt.-% by HPLC) were molten at 80 °C and 21.2 g polypropylene glycol (Pluriol P 600) were added. Approximately 310 g of the mixture were distilled by thin film evaporation (0.016 m², 450 U/min) at 5 mbar and 156 °C (jacket temperature). The mixture was added via pump (250 g/h). Except for the product collection all parts in contact with DEMAP including the condenser were heated to 80 °C. After distillation 281.8 g DEMAP (97.7 wt.-%, HPLC) were obtained as a yellow melt (sump: 24.6 g).

### General step 3: Purification of 2-(4'-Diethylamino-2'-hydroxybenzoyl)benzoic acid hexyl ester (DHHB)

The adsorption was done using a column with a diameter of 3.6 cm. The volume of the adsorbent (silica, activated carbon (also referred to as charcoal) or both) was always kept the same (height of adsorbent ∼10 cm). The column was packed with a slurry of the adsorbent in toluene or with the adsorbent followed by flushing with toluene before start of the adsorption.

The crystalline 2-(4'-Diethylamino-2'-hydroxybenzoyl)benzoic acid hexyl ester (hexanol-wet, -15% hexanol) prepared according to general step 2 was dissolved in toluene and purified by adsorption on silica and/or active charcoal. The concentration of the solution was 30% DHHB (cal. 100%). In total 100 g DHHB (cal. 100%) as solution in toluene (and residual hexanol) were added to the column in portions a 33.4 g (10 g DHHB cal. 100%. -38 ml of the solution). The product was collected in fractions. A new fraction was started each time when a new portion was started, only the first two fractions were collected together. In total 9 fractions were collected. The time for the addition of one fraction was -40 min. Each fraction was concentrated at 50 °C until a pressure of 4 mbar and further dried for 30 min at < 1 mbar (RT). For evaluation of the different parameters used in the synthesis, color numbers of comparable fractions were determined (criteria: total g ester after adsorption cal. 100%).

### Examples on adsorption (specific procedure of general step 3)

### IE1: (Table 2, No. 1): Purification 2-(4'-Diethylamino-2'-hydroxybenzoyl)benzoic acid hexyl ester

A column (∅ 3.6 cm) was packed with 1) 1 cm sand, 2) 5.4 g Silica 123 (Grace), 5.2 g Norit C GRAN (Cabot Norit), 3) 37 g Silica 123, and 4) 1 cm sand (filter paper between each layer). The column was flushed with 100 g toluene (disposal).

125.4 g of the ester prepared in step 2 were dissolved in 226 g toluene yielding a solution of -30 wt.-% of the ester in a mixture of toluene and residual hexanol. The solution was added continuously to the column in 10 portions a 33.4 g (40 min / portion). Each time a new portion was started, a new fraction was collected except for the first two fractions, which were collected together, since the first fraction is needed to load the column. Each product fraction was concentrated at 50 °C until a pressure of 4 mbar was reached (membrane pump) and further dried for 30 min at < 1 mbar.

### Further experiments

Based on the general process described in general steps 1-3, some parameters were varied: i) DEMAP distillation, ii) pH during extraction, and iii) the adsorbent. The total volume of adsorbent was always kept as described in IE1 (total height of adsorbent(s) -10 cm; ∅ = 3.6 cm).

The examples IE 2, C3, and C4 were all synthesized according to general step 1 to 3 and only the parameters changed are described (Table 2).

IE 2 and C3, and C4: A sample of DHHB was synthesized according to general steps 1 and 2 without prior distillation of DEMAP.

For IE2, the pH in the extraction after esterification in general step 2 was set to pH = 11.5. After crystallization 122.4 g (83.7 wt.-%) of the crystalline product were dissolved in 219 g toluene and filtered through the column filled with 1) 1 cm sand, 2) 5.4 g Silica 123 (Grace), 5.2 g Norit C GRAN (Cabot Norit), 3) 37 g Silica 123 and 4) 1 cm sand (filter paper between each layer, column flushed with 100 g toluene). Fractions were collected and concentrated as described in IE1 (Table 1).

For C3 and C4, the pH in the extraction after esterification in general step 2 was set to pH = 7. After crystallization 133 g (76.0 wt.-%) of the crystalline product were dissolved in 204 g toluene and filtered through the column filled with 1) 1 cm sand, 2) 19 g Norit C GRAN (Cabot Norit; for experiment C3) or 61.0 g Silica 123 (Grace; for experiment C4), and 3) 1 cm sand (filter paper between each layer, column flushed with 100 g toluene). Fractions were collected and concentrated as described in IE1 (Table 1).

**Table 1. Collected fractions obtained from IE1, IE2, C3, and C4**

| Ex. | Fraction | 1 | 2 | 3 | 4 | 5 | 6 | 7 | 8 | 9 |
|---|---|---|---|---|---|---|---|---|---|---|
| IE1 | [g] after conc. | 6.9 | 7.6 | 8.9 | 9.7 | 10.1 | 10.0 | 10.4 | 10.3 | 10.4 |
| IE2 | [g] after conc. | 6.2 | 7.9 | 9.2 | 9.9 | 10.2 | 10.3 | 10.6 | 10.5 | 10.1 |
| C3 | [g] after conc. | 5.9 | 7.3 | 8.7 | 9.7 | 9.8 | 10.2 | 10.7 | 10.8 | 11.4 |
| C4 | [g] after conc. | 7.8 | 8.6 | 9.3 | 10.2 | 10.9 | 11.2 | 10.7 | 11.2 | 11.3 |

**Table 2. Color numbers of fraction 9 of IE1, IE2, C3, and C4; Rh. denotes Rhodamine, Rh. ester denotes Rhodamine ester, G. denotes Gardner value, H.-I. denotes Hess-Ives, and YI denotes Yellowness Index.**

| No. | DHHB Synthesis | | | | Sample | | Rhodamines | | Color | | |
|---|---|---|---|---|---|---|---|---|---|---|---|
| | DEMAP dist. | pH | silica | Charcoal | Fraction No. | Total eluted [g] | Rh. [ppm] | Rh. ester [ppm] | G. | H.-I. | YI |
| IE1 | + | 11.4 | + | + | 9 | 82 | <1 | <1 | 7.3 | 12.4 | 81 |
| IE2 | - | 11.5 | + | + | 9 | 83 | <1 | <1 | 7.5 | 14.8 | 82 |
| C3 | - | 7 | - | + | 9 | 76 | <1 | <1 | 7.9 | 19.3 | 89 |
| C4 | - | 7 | + | - | 9 | 83 | <1 | <1 | 8.2 | 29.2 | 100 |

As can be seen by Table 2, the inventive process provides DHHB having a reduced amount of color impurities.

## Claims

1. Process of manufacturing 2-(4'-diethylamino-2'-hydroxybenzoyl)benzoic acid hexyl ester comprising the steps of
a) reacting phthalic acid anhydride with 3-N,N-diethylaminophenol to obtain 2-(4'-diethylamino-2'-hydroxybenzoyl)benzoic acid;
b) reacting the 2-(4'-diethylamino-2'-hydroxybenzoyl)benzoic acid obtained in step a) with n-hexanol to obtain 2-(4'-diethylamino-2'-hydroxybenzoyl)benzoic acid hexyl ester;
c) adjusting the reaction mixture obtained in step b) to a pH of more than 7 followed by extracting at least once with an aqueous phase; and
d) adsorbing the dissolved 2-(4'-diethylamino-2'-hydroxybenzoyl)benzoic acid hexyl ester over at least two different adsorbents.

2. The process of manufacturing according to claim 1,
wherein the 3-N,N-diethylaminophenol applied in step a) is distilled prior reacting with the phthalic acid anhydride and/or
has a Gardner value of 20 or lower, a Yellowness Index of 80 or lower, and/or a Hess-Ives of 350 or lower.

3. The process of manufacturing according to any one of claims 1 to 2,
wherein the pH of the reaction mixture in the extraction step c) is adjusted with an aqueous alkaline solution.

4. The process of manufacturing according to any one of claims 1 to 3,
wherein the pH of the reaction mixture in extraction step c) is from 8 to 14, preferably from 9 to 13, and in particular from 10.5 to 12.5.

5. The process of manufacturing according to any one of claim 1 to 4,
wherein in step c) the reaction mixture obtained in step b) is adjusted to a pH or more than 7 by the addition of a base to the reaction mixture obtained in step b).

6. The process of manufacturing according to any one of claims 1 to 5, further comprising step
cll) crystallizing the 2-(4'-diethylamino-2'-hydroxybenzoyl)benzoic acid hexyl ester from the organic phase obtained in the extraction step c).

7. The process of manufacturing according to claim 6,
wherein in the crystallization step cll), the crystallized 2-(4'-diethylamino-2'-hydroxybenzoyl)benzoic acid hexyl ester is dissolved in an organic solvent prior to the adsorption step d), preferably wherein the crystallized 2-(4'-diethylamino-2'-hydroxybenzoyl)benzoic acid hexyl ester is dissolved in a non-polar solvent, more preferably in toluene.

8. The process of manufacturing according to any one of claims 1 to 7,
wherein the at least two different adsorbents are selected from the group consisting of activated carbons, aluminum oxides, zeolites, and silica gels, preferably activated carbons and silica gels.

9. The process of manufacturing according to any one of claims 1 to 8,
wherein in the adsorption step d) the dissolved 2-(4'-diethylamino-2'-hydroxybenzoyl)benzoic acid hexyl ester is first adsorbed over silica gel and then adsorbed over activated carbon.

10. The process of manufacturing according to any one of claims 1 to 9,
wherein the solution in adsorption step d) comprises 2-(4'-diethylamino-2'-hydroxybenzoyl)benzoic acid hexyl ester from 10 to 80 wt.-%, preferably from 20 to 60 wt.-%, and in particular from 25 to 40 wt.-%, based on the total weight of the solution in adsorption step d).

11. The process of manufacturing according to any one of claims 1 to 10,
wherein the esterification step b) is carried out in the presence of an acidic catalyst, preferably wherein the acidic catalyst is sulfuric acid.

12. The process of manufacturing according to any one of claims 1 to 11, further comprising the step of
e) removing the solvent, preferably wherein the solvent is removed by distillation.

13. The process of manufacturing according to any one of claims 1 to 12,
wherein the manufactured 2-(4'-diethylamino-2'-hydroxybenzoyl)benzoic acid hexyl ester has a Gardner value of 8.2 or less, a Yellowness Index of less than 89, a Hess-Ives of less than 19, and/or comprises less than 5 ppm, preferably less than 2 ppm, and in particular less than 1 ppm, of rhodamine and its esters.

14. 2-(4'-Diethylamino-2'-hydroxybenzoyl)benzoic acid hexyl ester having a Gardner value of 8.2 or less, preferably less than 7.9, and in particular less than 7.5, and comprising less than 5 ppm, preferably less than 2 ppm, and in particular less than 1 ppm, of rhodamine and its esters, preferably wherein the 2-(4'-diethylamino-2'-hydroxybenzoyl)benzoic acid hexyl ester is obtained by the process according to any one of claims 1 to 13.

15. The 2-(4'-diethylamino-2'-hydroxybenzoyl)benzoic acid hexyl ester according to claim 14, wherein the 2-(4'-diethylamino-2'-hydroxybenzoyl)benzoic acid hexyl ester further has a Yellowness Index of less than 89, preferably less than 86 and/or a Hess-Ives of less than 19, preferably less than 17.
